# EUROPEAN PATENT SPECIFICATION

(11) **EP 2 493 838 B1**
(45) Date of publication and mention of the grant of the patent: **03.09.2014**
(21) Application number: 09748841.5
(22) Date of filing: 29.10.2009
(51) Int. Cl.: C01B 15/029, C07C 29/48, C07C 45/33

(54) **SELECTIVE CATALYTIC OXIDATION OF C1-C3 ALKANES**
SELEKTIVE KATALYTISCHE OXIDATION VON C1-C3 ALKANEN
OXIDATION CATALYTIQUE SELECTIVE DE C1-C3 ALKANE

(43) Date of publication of application: 05.09.2012
(73) Proprietor: IMPERIAL COLLEGE OF SCIENCE, TECHNOLOGY & MEDICINE, London SW7 2AZ (GB)
(72) Inventor: CHADWICK, David, Twickenham TW1 4RP (GB); MURCIANO, Laura, T., London SW11 4QU (GB)
(74) Representative: Beck Greener
(86) International application number: PCT/GB2009/051462
(87) International publication number: WO 2011/051643

(56) References cited:
- WO-A1-2008/108398
- GB-A- 1 041 046
- US-A1- 2003 073 573
- US-A1- 2003 134 741
- US-A1- 2004 138 480
- US-A1- 2006 293 175
- M.D.HUGHES ET AL: "Tunable gold catalysts for selective hydrocarbon oxidation under mild conditions" NATURE, 2005, pages 1132-1135, XP009136486

## Description

The invention relates to a process for oxidation of lower alkanes. More particularly, the invention relates to a process for oxidation of C₁-C₃ alkanes under mild conditions, using a heterogeneous catalyst on a metal oxide support having a particular morphology.

Today, both the chemical and energy industries rely on petroleum as the principal source of carbon and energy. Methane, a primary constituent of natural gas, which is an abundant carbon resource, offers great potential as a chemical feedstock, but has been underutilized. This is, in part, because it is thermodynamically and kinetically stable, that is, it is less reactive relative to many higher order alkanes. Methane's main industrial use is the production of synthesis gas (syngas) via stream reforming at high temperatures and pressures. Syngas can, however, be converted to methanol, which is much more reactive than methane and may be used as a starting material to prepare a wide variety of compounds and materials, including, for example, olefins. Unfortunately, the syngas route is energy intensive and therefore expensive, and also has undesirable environmental effects. A better route to methanol production has therefore been sought.

Selective oxidation of methane has been studied for over 30 years by individual, academic and government researchers with little or no commercial success. Direct methane oxidation processes are challenged by methane's general inertness, to both intermediates and to oxygenate products, and the difficulties of designing a catalytic process for a direct gas phase reaction with high conversion and selectivity. For example, Sen et al., in New J. Chem., 1989, 13, 755-760 disclose the use of Pd(O₂C Me)₂ in trifluoroacetic acid for the oxidation of methane to CF₃CO₂Me, wherein Me is the methanol constituent. The reaction is carried out for 4 days at a pressure of 5516-6895 kilopascals (kPa) (800-1000 pounds per square inch, psi). E. D. Park et al, in Catalysis Communications, Vol. 2 (2001), 187-190, disclose a Pd/C plus Cu(CH₃COO)₂ catalyst system for the selective oxidation of methane using hydrogen peroxide (H₂O₂). L.C. Kao et al., in J. Am. Chem. Soc., 113 (1991), 700-701 disclose the use of palladium compounds such as Pd(O₂CC₂H₅)₂ to oxidize methane to methanol in the presence of H₂O₂ and using trifluoroacetic acid as the solvent. U.S. Patent 5,585,515 discloses the use of catalysts such as copper iodide (Cu(I)) ions in trifluoroacetic acid to oxidize methane to methanol.

A problem that is encountered in many such processes is that high temperatures and/or pressures may destroy or alter products, while many processes tend to generate, in addition to the desired oxygenate product, a carbon oxygenate, such as carbon monoxide (CO) or carbon dioxide (CO₂), which compromises conversion rates and undesirably affects the environment. Inclusion of corrosive acids may create waste disposal problems. Attempts to counteract these difficulties have included liquid phase processes.

Recently it has been shown by Qiang Yuan et al., Adv. Synth. Catal. 349 (2007) 1199, that it is possible to oxidize methane in an aqueous medium using metal chlorides and H₂O₂, wherein the catalytic system is based on the use of hydrogen peroxide in water using homogeneous transition metal chlorides (for example, FeCl₃, CoCl₂. RuCl₃, RhCl₃, PdCl₂, OsCl₃, IrCl₃, H₂PtCl₆, CuCl₂, and HAuCl₄). This method has several process disadvantages, which include the fact that the homogeneous catalysts are highly soluble in water and therefore are difficult to separate for recycle purposes. Furthermore, as with some earlier processes, these homogeneous catalysts tend to show undesirable selectivities toward highly oxidized carbon species, such as formic acid and carbon dioxide (CO₂).

US2003/134741 discloses hydro-oxidation catalysts for the oxidation of hydrocarbons, containing an organic-inorganic hybrid material as well as gold particles.

Despite the multitude of methods for conversion of methane to methanol, there remains a need for a highly efficient and low cost process to accomplish the desired oxidation with fewer associated problems or drawbacks.

In one embodiment, the invention provides a process for preparing an oxygenate product from an alkane, comprising contacting a C₁-C₃ alkane and hydrogen peroxide or a hydroperoxy species, in the presence of a gold-based heterogeneous catalyst on a metal oxide support having a morphology of nanotubes, nanofibers, nanowires, or nanorods, under conditions such that an oxygenate product of the C₁-C₃ alkane is formed, wherein the metal oxide support having a morphology of nanotubes, nanofibers, nanowires, or nanorods includes an oxide selected from titanates (H_{z}Ti_{y}Oₓ), titania (TiO₂), cerias (H_{z}Ce_{y}Oₓ), zirconias (H_{z}Zr_{y}Oₓ), vanadias (H_{z}V_{y}Oₓ), aluminum oxide (Al₂O₃), silicon dioxide (SiO₂), and combinations thereof, wherein x represents an integer from 2 to 9, y represents an integer from 1 to 4, and z represents an integer from 0 to 3, depending upon the valence of the metal(s) in each given formula, and wherein sodium (Na) or potassium (K) may be substituted for hydrogen (H).

In another embodiment, the invention provides a process for preparing hydrogen peroxide or a hydroperoxy species comprising reacting a hydrogen source and an oxygen gas, in the presence of a gold-based heterogeneous catalyst on a metal oxide support having a morphology of nanotubes, nanofibers, nanowires, or nanorods, under conditions such that hydrogen peroxide or a hydroperoxy species are formed, wherein the metal oxide support having a morphology of nanotubes, nanofibers, nanowires, or nanorods includes an oxide selected from titanates (H_{z}Ti_{y}Oₓ), titania (TiO₂), cerias (H_{z}Ce_{y}Oₓ), zirconias (H_{z}Zr_{y}Oₓ), vanadias (H_{z}V_{y}Oₓ), aluminum oxide (Al₂O₃), silicon dioxide (SiO₂), and combinations thereof, wherein x represents an integer from 2 to 9, y represents an integer from 1 to 4, and z represents an integer from 0 to 3, depending upon the valence of the metal(s) in each given formula, and wherein sodium (Na) or potassium (K) may be substituted for hydrogen (H).

In a third embodiment, the invention provides a process for preparing an oxygenate product from an alkane comprising the steps of (1) reacting a hydrogen source and an oxygen gas to form a first product comprising hydrogen peroxide or a hydroperoxy species; and (2) reacting a C₁-C₃ alkane with the hydrogen peroxide or the hydroperoxy species to form a second product comprising an oxygenate product of the C₁-C₃ alkane, provided that both steps are carried out in the presence of a gold-based heterogeneous catalyst on a metal oxide support having a morphology of nanotubes, nanofibers, nanowires, or nanorods, under conditions such that the first product and the second product are formed, wherein the metal oxide support having a morphology of nanotubes, nanofibers, nanowires, or nanorods includes an oxide selected from titanates (H_{z}Ti_{y}Oₓ), titania (TiO₂), cerias (H_{z}Ce_{y}Oₓ), zirconias (H_{z}Zr_{y}Oₓ), vanadias (H_{z}V_{y}Oₓ), aluminum oxide (Al₂O₃), silicon dioxide (SiO₂), and combinations thereof, wherein x represents an integer from 2 to 9, y represents an integer from 1 to 4, and z represents an integer from 0 to 3, depending upon the valence of the metal(s) in each given formula, and wherein sodium (Na) or potassium (K) may be substituted for hydrogen (H).

In a fourth embodiment, the invention provides a process for preparing methanol from methane comprising the sequential steps of (1) reacting hydrogen and oxygen gas to form a first product comprising hydrogen peroxide or a hydroperoxy species; and (2) reacting methane with the hydrogen peroxide or the hydroperoxy species to form a second product comprising methanol, provided that both steps are carried out in the presence of a gold-based heterogeneous catalyst on a metal oxide support having a morphology of nanotubes, nanofibers, nanowires, or nanorods, under conditions such that the first product and the second product are formed, wherein the metal oxide support having a morphology of nanotubes, nanofibers, nanowires, or nanorods includes an oxide selected from titanates (H_{z}Ti_{y}Oₓ), titania (TiO₂), cerias (H_{z}Ce_{y}Oₓ), zirconias (H_{z}Zr_{y}Oₓ), vanadias (H₂V_{y}Oₓ), aluminum oxide (Al₂O₃), silicon dioxide (SiO₂), and combinations thereof, wherein x represents an integer from 2 to 9, y represents an integer from 1 to 4, and z represents an integer from 0 to 3, depending upon the valence of the metal(s) in each given formula, and wherein sodium (Na) or potassium (K) may be substituted for hydrogen (H).

The invention enables direct oxidative conversion of a C₁-C₃ alkane to an oxygenate product, which in some embodiments may be an alcohol, under mild conditions and using a specific catalyst and catalyst support of the structure defined hereinbelow. The result may be a high conversion rate in an inexpensive and commercially viable process.

The basic oxidative conversion may be accomplished by contacting a C₁-C₃ alkane, such as methane, with hydrogen peroxide (H₂O₂) or a hydroperoxy species that are, in one embodiment, in aqueous solution. As used herein the term "C₁-C₃ alkane" refers to, respectively, methane, ethane, propane, or a combination thereof, and the term "a hydroperoxy species" refers to any O₂H radical or any organic or inorganic compound containing an O₂H functional group or radical. The contact with the hydrogen peroxide or the hydroperoxy species efficiently oxidizes the selected alkane, for example, methane, to form an oxygenate product, for example, methanol, without over-oxidation which may result in formation of undesirable carbon oxygenates, formaldehyde, formic acid, and/or water, which in turn result in a lower methanol yield. A key to the excellent selectivity and efficiency of the reaction is that this contact is carried out in the presence of a gold-based (Au-based) heterogeneous catalyst, which is generally composed of catalyst particles which are deposited onto the surface of a metal oxide support having a morphology of nanotubes, nanofibers, nanowires, or nanorods. By heterogeneous is meant that the solid catalyst is clearly not being solubilized in either the liquid (aqueous solution) phase or, in another embodiment, in the gas phase. "Liquid phase" refers, for purposes hereof, to any material or combination of materials that is a liquid under the process conditions, and "gas phase" refers to any material or combination of materials that is a gas under the process conditions. Such catalyst may include gold itself and its combination with additional metals, as alloys. Such additional metals may include, for example, palladium (Pd) and platinum (Pt), which serve to increase the activity of the gold. Other metals which may be alloyed with gold for use herein include, for example, silver (Ag), copper (Cu), rhodium (Rh), iridium (Ir), ruthenium (Ru), or combinations thereof. These alloys may be ordered or disordered and may additionally show surface segregation effects wherein one component is preferentially enriched at the catalyst particle surface or the catalyst particle/oxide support interface. These may be further modified by use of a suitable promoter to enhance selectivity, for example, a halide, such as a chloride; a phosphate; an alkali metal, such as sodium or potassium; or a combination thereof.

The catalysts generally contain gold in amounts from preferably 0.001 to 10 percent by weight based on the total weight of the catalyst, more preferably in the range of 0.1 to 5 percent by weight, and most preferably in the range of 0.2 to 2 percent. In preferred embodiments, where palladium is also included, the palladium is preferably in amounts from 0.001 to 10 percent by weight based on the total weight of the catalyst, more preferably in the range of from 1 to 5 percent by weight, and most preferably in the range of from 1 to 3 percent. In certain other embodiments, the catalyst may alternatively include a metal other than, or in addition to, palladium, along with the gold. In that case it is preferred that the amount of the additional metal range from 0.001 to 10 percent by weight, more preferably from 1 to 5 percent, and most preferably from 2 to 3 percent. If other metals such as promoters are present, they are typically in an amount of from 0.001 to 5 percent by weight, based on the weight of the total catalyst.

The amount of total catalyst employed in a given reaction can vary widely. The catalyst can be used in any amount that provides conversion (oxidation) of at least a portion of the C₁-C₃ alkane to be converted. It is possible to employ two or more catalysts in the practice of this invention, which may be selected in order to achieve a specific result that is unachievable with a single catalyst.

The oxide support used in the invention is characterized as having a support particle morphology of nanotubes, nanofibers, nanowires, or nanorods. These support particle structures are defined herein as being configured of one or more individual components characterized as having at least one dimension ranging from 1 to 100 nanometers (nm). The structures may be of various configurations, such as, for example, tubes (including but not limited to rings), wires, fibers, or rods. The nanotubes (open or capped), nanofibers, nanowires, or nanorods may be characterized as having walls composed of either an amorphous or an ordered crystal structure having negligible porosity. As used herein, the term "negligible" refers to porosity which is considered to be chemically irrelevant to the catalysis taking place in the invention. Furthermore, support particles may self-assemble into larger bodies while retaining the morphology of the individual components. The nanotubular, nanofibral, nanowire, or nanorod oxide supports may be composed of any suitable metal oxide, including but not limited to those metal oxides that are frequently categorized as constituting ceramics or other refractory materials. Such may include, but are not limited to, titanates (H_{z}Ti_{y}Oₓ), titania (TiO₂), cerias (H_{z}Ce_{y}Oₓ), zirconias (H_{z}Zr_{y}Oₓ), vanadias (H_{z}V_{y}Oₓ), aluminum oxide (Al₂O₃), silicon dioxide (SiO₂), and combinations thereof, wherein x represents an integer from 2 to 9, y represents an integer from 1 to 4, and z represents an integer from 0 to 3, depending upon the valence of the metal(s) in each given formula. Alkali metals such as sodium (Na) or potassium (K) may be introduced into the chemical composition by replacement of hydrogen ions or due to residual traces of these metals that may be present during the synthesis.

These structures may be prepared by any means or method known to those skilled in the art of preparing such supports. For example, a nanotubular titanate support (H₂Ti₃O₇) may be used in certain embodiments. Preparation of that support may be accomplished by, for example, hydrothermal treatment of TiO₂ in sodium hydroxide (NaOH), followed by hydrogen chloride (HCl) washing to remove sodium (Na). This procedure may result in the nanotube configuration. Washing is followed by calcination at, for example, 400 degrees Celsius (°C). Other methods are summarized in, for example, Y.X. Xia et al., Adv. Materials 15 (2003) 353, the entirety of which is incorporated herein by reference. However, the hydrothermal method is preferred due to the greater control it provides in determining the oxide support's final structure.

Various methods may be employed in order to apply the gold-based heterogeneous catalyst to the oxide nanotubes, nanofibers, nanowires, or nanorods. These methods may include, in non-limiting embodiments, ion exchange, impregnation to incipient wetness, deposition precipitation (with or without urea), sol immobilization, and chemical vapor deposition. For example, in one embodiment a 0.6 percent gold (Au)/1.5 percent palladium (Pd) catalyst may be synthesized on the external surface of a nanotubular oxide support by concurrent or sequential ion exchange with the metal salts HAuCl₄ and PdCl₂. This ion exchange process may result in gold-palladium particles having a narrow particle size distribution ranging from 2 nm to 5 nm. The particle size and distribution are independent of total metal loading.

### Methane Oxidation Reaction (Liquid Phase)

In order to carry out one non-limiting embodiment of the invention, methane may be bubbled through an aqueous solution of hydrogen peroxide in the presence of a suspended gold-based heterogeneous catalyst on a metal oxide support having a morphology of nanotubes, nanofibers, nanowires, or nanorods. Stirring is desirable in order to maximize contact between the methane, the hydrogen peroxide, and the catalyst, but is not a requirement of the invention. The methane may be fed to the reaction, preferably at partial pressures ranging from 0.1 atmosphere (atm) (1.01 x 10⁴ newtons per square meter, N/m²) to 140 atm (1.42 x 10⁷ N/m²), more preferably ranging from 0.2 atm (0.20 x 10⁵ N/m²) to 100 atm (1.01 x 10⁷ N/m²), and most preferably from 0.5 atm (0.51 x 10⁵ N/m²) to 70 atm (7.10 x 10⁶ N/m²). In particularly preferred embodiments, the aqueous solution may be maintained at a temperature ranging from 0°C to 200°C, more preferably less than 100°C, and still more preferably from 30°C to 90°C. For example, it has been found that the heterogeneous gold-based catalysts can oxidize methane to methanol in water using hydrogen peroxide at temperatures as low as 30°C, circumventing the need for high temperatures to activate methane such as occurs in other methods where selectivity losses to carbon oxygenates, such as CO and/or CO₂, are observed. This reaction yields methanol and water, and the methanol may be subsequently separated using any of a variety of known separation techniques such as fractional distillation, selective adsorption, or liquid-liquid extraction.

Because the catalysts are not soluble in the liquid, they can be recovered by standard separation techniques and then recycled for subsequent use in another reaction. If the catalyst loses activity over time, standard regeneration techniques may be used to reactivate it, such as by burning off build-up on the catalyst or treating the catalyst with fresh hydrogen peroxide solutions. Alternatively, fresh catalyst may be introduced.

### Ethane and Propane Oxidation Reactions (Liquid Phase)

Similar to the oxidation of methane, ethane may be oxidized using the process of the invention as described hereinabove. In this case the resulting oxygenate product will be ethanol. Again, selectivity of oxygenate products of carbon, including CO and/or CO₂, is not generally encountered. Propane may also be oxidized in a similar manner, except that it may be simply fed into the aqueous H₂O₂ solution rather than bubbled through it if the propane is in a liquid phase. The resulting oxygenate product will be isopropanol, with essentially no carbon oxygenate products.

### Combined Hydrogen Oxidation and Methane Oxidation

While aqueous solutions of hydrogen peroxide can be used directly, in another embodiment of the invention hydrogen peroxide and/or a hydroperoxy species may be generated in an aqueous medium *in situ,* from a hydrogen source and an oxygen gas. By *in situ* is meant that the hydrogen peroxide and/or the hydroperoxy species are produced either simultaneously with the oxidation of the C₁-C₃ alkane in a single reactor, or that the two reactions are sequenced in a single reactor, using, for example, an intermittently operated fixed-bed reactor, a monolith reactor, a slurry reactor, or a moving bed reactor. Sequencing may also be carried out in a continuous flow reactor having, for example, multiple sequential fixed catalyst beds, or in a continuous flow reactor system with multiple stages. Any source of hydrogen can be used in the process of this invention, including hydrogen gas or, for example, molecular hydrogen obtained from the dehydrogenation of hydrocarbons and alcohols. The source of oxygen, however, desirably includes oxygen gas *per se,* and may include air, for example, or pure oxygen.

It is generally desirable that the amount of hydrogen and of oxygen gas is sufficient to produce hydrogen peroxide and/or a hydroperoxy species in the desired quantities to match the rate of alkane conversion.

To carry out both oxidation reactions, an aqueous medium containing the catalyst or catalysts may be first placed in a suitable reactor. When a closed reactor is used, the hydrogen source/oxygen gas mixture may be mixed with methane and an optional diluent and pressurized up to a total pressure preferably ranging from 1 atm (1.01 x 10⁵ N/m²) to 140 atm (1.42 x 10⁷ N/m²), more preferably from 5 atm (5.05 x 10⁵ N/m²) to 100 atm (1.01 x 10⁷ N/m²), and most preferably from 10 atm (1.01 x 10⁶ N/m²) to 70 atm (7.10 x 10⁶ N/m²). Preferably a ratio of H₂:O₂ ranging from 1:5 to 5:1, with optional diluent, is useful for forming the *in situ* hydrogen peroxide and/or the hydroperoxy species. More preferably the H₂:O₂ ratio ranges from 1:3 to 3:1, and most preferably the H₂:O₂ ratio ranges from 1:2 to 2:1. It is advisable to employ H₂:O₂ ratios with appropriate alkane and diluent pressure to avoid using explosive mixtures. The reaction is preferably maintained at a temperature from 0°C to 200°C, more preferably from 10°C to 100°C, and most preferably from 30°C to 90°C.

In alternative embodiments, the hydrogen oxidation may be carried out in one step, and the C₁-C₃ oxidation in a second step, simply by sequencing the flow of reactants to the reactor. This can be done with either solely gas-phase reactants and products and a solid catalyst, or with gaseous reactants and a catalyst suspended in a suitable solvent wherein the products are in the liquid phase. Intermittent reactor operation may be necessary to enable both appropriate periodic removal of generated methanol and also catalyst regeneration. Where the reaction is carried out in the two-step sequence, methane will convert to methanol, but ethane and propane may, depending upon conditions, convert to a wider range of oxygenate products than only ethanol and isopropanol, respectively. Some of these oxygenate products are not alcohols. For example, formic acid may be a by-product of ethane or propane oxidation.

### Example 1

Titanate nanotubes are synthesized following a hydrothermal method described in detail in Bavykin, D.V., Parmon, V.N., Lapkin, A.A., Walsh, F.C., J. Mater. Chem., 14 (2004) 3370. A 0.6 percent Au/1.5 percent Pd catalyst is synthesized by ion exchange with the metal salts HAuCl₄ and PdCl₂. Al₂O₃, SiO₂ and TiO₂ supports are obtained from commercial sources. The catalysts are deposited on the supports by wet impregnation using the metal salts HAuCl₄ and PdCl₂. These catalysts are pre-reduced under a hydrogen (H₂) flow at 200°C for 90 minutes.

Catalyst testing is carried out in a Parr autoclave (250 mL) with a glass liner and gas aspirating stirrer using a mixed gas of methane/helium/argon (CH₄/He/Ar, 5 percent/2.5 percent/92.5 percent) and an aqueous solution of hydrogen peroxide. Catalyst, in the amount of 0.02 gram (g), is added to 75 milliliters (mL) of analytical grade water containing 0.037 mole H₂O₂. Reaction temperature is 30°C. Reaction time is 0.5 hr. Liquid product analysis is done by gas chromatography (GC). Gas samples are taken from the head space at the end of the reaction and analysis for CO₂ is by GC. The results are given in Table 1. No CO₂ is detected.

**Table 1**

| Catalyst | Total Pressure (bar) | Methanol (micromoles) | Catalyst Activity (mol/kg cat h)** |
|---|---|---|---|
| Au-Pd/Ti-nt* | 30** | 40.7 | 4.1 |
| Au-Pd/TiO₂ | 30 | 22.2 | 2.2 |
| Au-Pd/SiO₂ | 30 | 25.6 | 2.5 |
| Au-Pd/Al₂O₃ | 30 | 17.9 | 1.8 |

| | | | |
|---|---|---|---|
| *nt is nanotubes **30 bar equals 3.00 x 10⁶ N/m² ***mol/kg cat h is moles methanol per kilogram of catalyst per hour) | | | |

### Example 2

Catalysts are prepared as in Example 1, and tested in accord with that Example to show the effect of pressure on yield and catalyst efficiency. Results are shown in Table 2. No CO₂ or formic acid is detected.

**Table 2**

| Catalyst | Total Pressure (bar) | Methanol (micromoles) | Catalyst Activity (mol/kg-cat h) |
|---|---|---|---|
| Au-Pd/Ti-nt | 30* | 40.7 | 4.1 |
| Au-Pd/Ti-nt | 10** | 128.1 | 12.8 |
| Au-Pd/TiO₂ | 30 | 22.2 | 2.2 |
| Au-Pd/TiO₂ | 10 | 56.2 | 5.6 |

| | | | |
|---|---|---|---|
| *30 bar equals 3.00 x.10⁶ N/m² **10 bar equals 1.00 x 10⁶ N/m² | | | |

### Example 3

Titanate nanotubes are synthesized according to Example 1. Catalyst testing is then carried out in a fixed-bed microflow reactor with 0.2 g of catalyst at atmospheric pressure. Reaction temperature is 60°C. The catalyst is exposed to a flow of 100 milliliters per minute (mL/min) of a 5 percent H₂/10 percent O₂ mixture in nitrogen (N₂) for 5 minutes to cover the catalyst surface in a hydroperoxy species or adsorbed hydrogen peroxide. Afterward, the H₂ and O₂ are swept out by a flow of 100 mL/min argon (Ar). Then a pulse of 20 mL of a gas mixture of CH₄/He/Ar (5 percent/2.5 percent/92.5 percent) is injected into the Ar and passed through the catalyst bed. Product gas is passed through a water trap in an ice bath and samples of the water are taken for analysis by GC. The results are given in Table 3.

**Table 3**

| Catalyst | Weight (g) | Methanol (micromoles) | Catalyst Activity* (mol/kg-cat h) |
|---|---|---|---|
| Au-Pd/Ti-nt | 0.2 | 27 | 271 |

| | | | |
|---|---|---|---|
| *Catalyst Activity estimated from the amount of methanol and the flow rate, pulse duration and number of pulses. | | | |

### Example 4

Catalyst preparation is as described in Example 3. Catalyst testing is carried out using the apparatus of Example 3. The catalyst is exposed to a flow of 100 mL/min of 5 percent H₂/10 percent O₂ in N₂ for 5 minutes to cover the catalyst surface in a hydroperoxy species or adsorbed hydrogen peroxide. Afterward, H₂ and O₂ are swept out by a flow of Ar. Then a continuous flow of 20 mL/min of a gas mixture of CH₄/He/Ar (5 percent/2.5 percent/92.5 percent) is passed through the catalyst bed. Product gas is passed through a water trap in an ice bath and samples of the water are taken for analysis by GC. Results are in Table 4.

**Table 4**

| Catalyst | Weight (g) | Methanol (micromoles) | Catalyst Activity (mol/kg-cat h) |
|---|---|---|---|
| Au-Pd/Ti-n | 0.2 | 21 | 213 |

### Example 5

Catalyst preparation is as described in Example 3. Catalyst testing is carried out in a fixed-bed microflow reactor with 0.2 g of catalyst at atmospheric pressure. Reaction temperature is 60°C. The catalyst is exposed to a flow of 100 mL/min of 5. percent H₂/10 percent O₂ in N₂ for 5 minutes to cover the catalyst surface in a hydroperoxy species or adsorbed hydrogen peroxide. Afterward, H₂ and O₂ are swept out by a flow of 100 mL/min Ar. Then a pulse of 10 mL of a gas mixture of CH₄/He/Ar (5 percent/2.5 percent/92.5 percent) is injected into the Ar and passed through the catalyst bed. Product gas is analyzed on-line by mass spectrometer for methane and CO₂. The gold-palladium heterogeneous catalyst on a titanate nanotubular support (Au-Pd/Ti-nt) shows the highest methane conversion (ranging from 40 to 70 percent). Methanol is detected but not quantified. No CO₂ is detected in the product gas.

### Example 6

Catalysts are prepared according to Example 1 and tested according to that Example, except that the gas being oxidized is 5 percent ethane in an inert gas. Ethane is oxidized to ethanol. No other by-products are detected. No CO₂ or formic acid is detected. The results are given in Table 5.

**Table 5**

| Catalyst | Total Pressure (bar) | Ethanol (micromoles) | Catalyst Activity (mol/kg-cat h) |
|---|---|---|---|
| Au-Pd/Ti-nt | 8.5* | 358 | 35.8 |
| Au-Pd/TiO₂ | 8.5 | 25 | 2.5 |

| | | | |
|---|---|---|---|
| *8.5 bar equals 8.5 x 10⁵ N/m² | | | |

### Example 7

Catalysts are prepared according to Example 1 and tested according to that Example, except that the gas being oxidized is 10 percent propane in an inert gas. Propane is oxidized to 2-propanol. No other by-products are detected. No CO₂ or formic acid is detected. The results are given in Table 6.

**Table 6**

| Catalyst | Total Pressure (bar) | 2-Propanol (micromoles) | Catalyst Activity (mol/kg-cat h) |
|---|---|---|---|
| Au-Pd/Ti-nt | 8.5* | 9.9 | 1.0 |
| Au-Pd/TiO₂ | 8.5 | 6.3 | 0.6 |

| | | | |
|---|---|---|---|
| *8.5 bar equals 8.5 x 10⁵ N/m² | | | |

## Claims

1. A process for preparing an oxygenate product from an alkane, comprising contacting a C₁-C₃ alkane and hydrogen peroxide or a hydroperoxy species, in the presence of a gold-based heterogeneous catalyst on a metal oxide support having a morphology of nanotubes, nanofibers, nanowires, or nanorods, under conditions such that an oxygenate product of the C₁-C₃ alkane is formed, wherein the metal oxide support having a morphology of nanotubes, nanofibers, nanowires, or nanorods includes an oxide selected from titanates (H_{z}Ti_{y}Oₓ), titania (TiO₂), cerias (H_{z}Ce_{y}Oₓ), zirconias (H_{z}Zr_{y}Oₓ), vanadias (H_{z}V_{y}Oₓ), aluminum oxide (Al₂O₃), silicon dioxide (SiO₂), and combinations thereof, wherein x represents an integer from 2 to 9, y represents an integer from 1 to 4, and z represents an integer from 0 to 3, depending upon the valence of the metal(s) in each given formula, and wherein sodium (Na) or potassium (K) may be substituted for hydrogen (H).

2. The process of Claim 1 wherein the gold-based heterogeneous catalyst includes an alloy of gold and at least one additional metal selected from palladium, platinum, silver, copper, rhodium, iridium, ruthenium, and combinations thereof.

3. The process of Claim 1 wherein the conditions include a temperature ranging from 0°C to 200°C and a partial alkane pressure ranging from 1.01 x 10⁴ newtons per square meter to 1.42 x 10⁷ newtons per square meter.

4. The process of claim 1 wherein the C₁-C₃ alkane is selected from methane, wherein the oxygenate product is methanol; ethane, wherein the oxygenate product is ethanol; and propane, wherein the oxygenate product is 2-propanol.

5. The process of Claim 1 further including a promoter selected from chloride, phosphate, sodium, potassium, and combinations thereof.

6. The process of Claim 1 wherein no carbon oxygenate product of the C₁-C₃ alkane is formed.

7. The process of Claim 1 wherein the hydrogen peroxide or the hydroperoxy species are first prepared in aqueous solution and the C₁-C₃ alkane then contacts the hydrogen peroxide or the hydroperoxy species in the presence of the gold-based heterogeneous catalyst, the hydrogen peroxide or the hydroperoxy species being either in the aqueous solution or adsorbed to the catalyst.

8. The process of Claim 1 wherein the hydrogen peroxide or hydroperoxy-species are prepared by reacting a hydrogen source and an oxygen gas, in the presence of a gold-based heterogeneous catalyst on a metal oxide support having a morphology of nanotubes, nanofibers, nanowires, or nanorods, under conditions such that hydrogen peroxide or a hydroperoxy species are formed.

9. The process of Claim 8 wherein the reacting of the hydrogen source and the oxygen gas to form hydrogen peroxide or a hydroperoxy species, and the contacting of the C₁-C₃ alkane and the hydrogen peroxide or the hydroperoxy species, are carried out as sequential steps in a single reactor or in a reactor system with multiple stages.

10. The process of Claim 9 wherein the single reactor is, or the reactor system includes, a fixed bed reactor, a monolith reactor, a slurry reactor, or a moving bed reactor.

11. The process of Claim 1 wherein either (a) the C₁-C₃ alkane and the oxygenate product are both gas-phase, and the catalyst is solid; or (b) the C₁-C₃ alkane is gas phase, the catalyst is suspended in a solvent, and the oxygenate product is liquid phase.

12. A process for preparing methanol from methane comprising the sequential steps of (1) reacting hydrogen and oxygen gas to form a first product comprising hydrogen peroxide or a hydroperoxy species; and (2) reacting methane with the hydrogen peroxide or the hydroperoxy species to form a second product comprising methanol, provided that both steps are carried out in the presence of a gold-based heterogeneous catalyst on a metal oxide support having a morphology of nanotubes, nanofibers, nanowires, or nanorods, under conditions such that the first product and the second product are formed, wherein the metal oxide support having a morphology of nanotubes, nanofibers, nanowires, or nanorods includes an oxide selected from titanates (H_{z}Ti_{y}Oₓ), titania (TiO₂), cerias (H_{z}Ce_{y}O_{z}), zirconias (H_{z}Zr_{y}Oₓ), vanadias (H_{z}V_{y}Oₓ), aluminum oxide (Al₂O₃), silicon dioxide (SiO₂), and combinations thereof, wherein x represents an integer from 2 to 9, y represents an integer from 1 to 4, and z represents an integer from 0 to 3, depending upon the valence of the metal(s) in each given formula, and wherein sodium (Na) or potassium (K) may be substituted for hydrogen (H).

13. The process of Claim 12 wherein, in step (a) and step (b), the hydrogen source, the oxygen gas, the methane, and the hydrogen peroxide or the hydroperoxy species are all in gas phase, and the catalyst is a solid.

14. A process for preparing hydrogen peroxide or a hydroperoxy species comprising reacting a hydrogen source and an oxygen gas, in the presence of a gold-based heterogeneous catalyst on a metal oxide support having a morphology of nanotubes, nanofibers, nanowires, or nanorods, under conditions such that hydrogen peroxide or a hydroperoxy species are formed, wherein the metal oxide support having a morphology of nanotubes, nanofibers, nanowires, or nanorods includes an oxide selected from titanates (H_{z}Ti_{y}Oₓ), titania (TiO₂), cerias (H_{z}Ce_{y}Oₓ), zirconias (H_{z}Zr_{y}Oₓ), vanadias (H_{z}V_{y}Oₓ), aluminum oxide (Al₂O₃), silicon dioxide (SiO₂), and combinations thereof, wherein x represents an integer from 2 to 9, y represents an integer from 1 to 4, and z represents an integer from 0 to 3, depending upon the valence of the metal(s) in each given formula, and wherein sodium (Na) or potassium (K) may be substituted for hydrogen (H).

## Patentansprüche

1. Ein Verfahren zum Zubereiten eines Oxygenatprodukts aus einem Alkan, beinhaltend das In-Kontakt-Bringen eines C₁-C₃-Alkans und Wasserstoffperoxid oder einer Hydroperoxyspezies in der Gegenwart eines goldbasierten heterogenen Katalysators auf einem Metalloxidträger mit einer Morphologie von Nanoröhrchen, Nanofasern, Nanodrähten oder Nanostäben unter Bedingungen, so dass ein Oxygenatprodukt des C₁-C₃-Alkans gebildet wird, wobei der Metalloxidträger mit einer Morphologie von Nanoröhrchen, Nanofasern, Nanodrähten oder Nanostäben ein Oxid umfasst, ausgewählt aus Titanaten (H_{z}Ti_{y}Oₓ), Titandioxid (TiO₂), Cerdioxiden (H_{z}Ce_{y}Oₓ), Zirconiumdioxiden (H_{z}Zr_{y}Oₓ), Vanadiumpentoxiden (H_{z}V_{y}Oₓ), Aluminiumoxid (Al₂O₃), Siliciumdioxid (SiO₂) und Kombinationen davon, wobei x eine ganze Zahl von 2 bis 9 darstellt, y eine ganze Zahl von 1 bis 4 darstellt, und z eine ganze Zahl von 0 bis 3 darstellt, abhängig von der Valenz des Metalls/der Metalle in jeder gegebenen Formel, und wobei Natrium (Na) oder Kalium (K) durch Wasserstoff (H) ersetzt werden kann.

2. Verfahren gemäß Anspruch 1, wobei der goldbasierte heterogene Katalysator eine Legierung aus Gold und mindestens einem zusätzlichen Metall, ausgewählt aus Palladium, Platin, Silber, Kupfer, Rhodium, Iridium, Ruthenium und Kombinationen davon, umfasst.

3. Verfahren gemäß Anspruch 1, wobei die Bedingungen eine Temperatur im Bereich von 0 °C bis 200 °C und einen Alkan-Partialdruck im Bereich von 1,01 x 10⁴ Newton pro Quadratmeter bis 1,42 x 10⁷ Newton pro Quadratmeter umfassen.

4. Verfahren gemäß Anspruch 1, wobei das C₁-C₃-Alkan aus Methan, wobei das Oxygenatprodukt Methanol ist; Ethan, wobei das Oxygenatprodukt Ethanol ist; und Propan, wobei das Oxygenatprodukt 2-Propanol ist, ausgewählt ist.

5. Verfahren gemäß Anspruch 1, das ferner einen Promotor umfasst, der aus Chlorid, Phosphat, Natrium, Kalium und Kombinationen davon ausgewählt ist.

6. Verfahren gemäß Anspruch 1, wobei kein Kohlenstoffoxygenatprodukt des C₁-C₃-Alkans gebildet wird.

7. Verfahren gemäß Anspruch 1, wobei das Wasserstoffperoxid oder die Hydroperoxyspezies zuerst in wässriger Lösung zubereitet werden und das C₁-C₃-Alkan dann das Wasserstoffperoxid oder die Hydroperoxyspezies in der Gegenwart des goldbasierten heterogenen Katalysators kontaktiert, wobei sich das Wasserstoffperoxid oder die Hydroperoxyspezies entweder in der wässrigen Lösung befindet oder an dem Katalysator angelagert ist.

8. Verfahren gemäß Anspruch 1, wobei das Wasserstoffperoxid oder die Hydroperoxyspezies durch das Reagieren einer Wasserstoffquelle und eines Sauerstoffgases zubereitet wird, in der Gegenwart eines goldbasierten heterogenen Katalysators auf einem Metalloxidträger mit einer Morphologie von Nanoröhrchen, Nanofasern, Nanodrähten oder Nanostäben, unter Bedingungen, so dass Wasserstoffperoxid oder eine Hydroperoxyspezies gebildet wird.

9. Verfahren gemäß Anspruch 8, wobei das Reagieren der Wasserstoffquelle und des Sauerstoffgases, um Wasserstoffperoxid oder eine Hydroperoxyspezies zu bilden, und das Kontaktieren des C₁-C₃-Alkans und des Wasserstoffperoxids oder der Hydroperoxyspezies als aufeinanderfolgende Schritte in einem einzelnen Reaktor oder in einem Reaktorsystem mit mehreren Stufen ausgeführt werden.

10. Verfahren gemäß Anspruch 9, wobei der einzelne Reaktor Folgendes ist oder das Reaktorsystem Folgendes umfasst: einen Festbettreaktor, einen Monolithreaktor, einen Suspensionsreaktor oder einen Bewegtbettreaktor.

11. Verfahren gemäß Anspruch 1 wobei entweder (a) das C₁-C₃-Alkan und das Oxygenatprodukt beide im gasförmigen Zustand sind und der Katalysator fest ist; oder (b) das C₁-C₃-Alkan im gasförmigen Zustand ist, der Katalysator in einem Lösungsmittel suspendiert ist und das Oxygenatprodukt im flüssigen Zustand ist.

12. Ein Verfahren zum Zubereiten von Methanol aus Methan, das die folgenden aufeinanderfolgenden Schritte beinhaltet: (1) Reagieren von Wasserstoff und Sauerstoffgas, um ein erstes Produkt zu bilden, das Wasserstoffperoxid oder eine Hydroperoxyspezies beinhaltet; und (2) Reagieren von Methan mit dem Wasserstoffperoxid oder der Hydroperoxyspezies, um ein zweites Produkt zu bilden, das Methanol beinhaltet, vorausgesetzt, dass beide Schritte in der Gegenwart eines goldbasierten heterogenen Katalysators auf einem Metalloxidträger mit einer Morphologie von Nanoröhrchen, Nanofasern, Nanodrähten oder Nanostäben unter Bedingungen, so dass das erste Produkt und das zweite Produkt gebildet werden, ausgeführt werden, wobei der Metalloxidträger mit einer Morphologie von Nanoröhrchen, Nanofasern, Nanodrähten oder Nanostäben ein Oxid umfasst, ausgewählt aus Titanaten (H_{z}Ti_{y}Oₓ), Titandioxid (TiO₂), Cerdioxiden (H_{z}Ce_{y}Oₓ), Zirconiumdioxiden (H_{z}Zr_{y}Oₓ), Vanadiumpentoxiden (H_{z}V_{y}Oₓ), Aluminiumoxid (Al₂O₃), Siliciumdioxid (SiO₂) und Kombinationen davon, wobei x eine ganze Zahl von 2 bis 9 darstellt, y eine ganze Zahl von 1 bis 4 darstellt, und z eine ganze Zahl von 0 bis 3 darstellt, abhängig von der Valenz des Metalls/der Metalle in jeder gegebenen Formel, und wobei Natrium (Na) oder Kalium (K) durch Wasserstoff (H) ersetzt werden kann.

13. Verfahren gemäß Anspruch 12, wobei sich in Schritt (a) und Schritt (b) die Wasserstoffquelle, das Sauerstoffgas, das Methan und das Wasserstoffperoxid oder die Hydroperoxyspezies alle in gasförmigem Zustand befinden, und der Katalysator ein Festkörper ist.

14. Ein Verfahren zum Zubereiten von Wasserstoffperoxid oder einer Hydroperoxyspezies. beinhaltend das Reagieren einer Wasserstoffquelle und eines Sauerstoffgases in der Gegenwart eines goldbasierten heterogenen Katalysators auf einem Metalloxidträger mit einer Morphologie von Nanoröhrchen, Nanofasern, Nanodrähten oder Nanostäben unter Bedingungen, so dass Wasserstoffperoxid oder eine Hydroperoxyspezies gebildet werden, wobei der Metalloxidträger mit einer Morphologie von Nanoröhrchen, Nanofasern, Nanodrähten oder Nanostäben ein Oxid umfasst, ausgewählt aus Titanaten (H_{z}Ti_{y}Oₓ), Titandioxid (TiO₂), Cerdioxiden (H_{z}Ce_{y}Oₓ), Zirconiumdioxiden (H_{z}Zr_{y}Oₓ), Vanadiumpentoxiden (H_{z}V_{y}Oₓ), Aluminiumoxid (Al₂O₃), Siliciumdioxid (SiO₂) und Kombinationen davon, wobei x eine ganze Zahl von 2 bis 9 darstellt, y eine ganze Zahl von 1 bis 4 darstellt, und z eine ganze Zahl von 0 bis 3 darstellt, abhängig von der Valenz des Metalls/der Metalle in jeder gegebenen Formel, und wobei Natrium (Na) oder Kalium (K) durch Wasserstoff (H) ersetzt werden kann.

## Revendications

1. Un procédé pour préparer un produit oxygéné à partir d'un alcane, comprenant la mise en contact d'un alcane en C₁ à C₃ et de peroxyde d'hydrogène ou d'une espèce hydroperoxy, en présence d'un catalyseur hétérogène à base d'or sur un support d'oxyde métallique ayant une morphologie de nanotubes, de nanofibres, de nanofils, ou de nanotiges, dans des conditions telles qu'un produit oxygéné de l'alcane en C₁ à C₃ soit formé, dans lequel le support d'oxyde métallique ayant une morphologie de nanotubes, de nanofibres, de nanofils, ou de nanotiges inclut un oxyde sélectionné parmi des titanates (H_{z}Ti_{y}Oₓ), le dioxyde de titane (TiO₂), des oxydes de cérium (H_{z}Ce_{y}Oₓ), des oxydes de zirconium (H_{z}Zr_{y}Oₓ), des oxydes de vanadium (H_{z}V_{y}Oₓ), l'oxyde d'aluminium (Al₂O₃), le dioxyde de silicium (SiO₂), et des combinaisons de ceux-ci, dans lequel x représente un nombre entier de 2 à 9, y représente un nombre entier de 1 à 4, et z représente un nombre entier de 0 à 3, en fonction de la valence du (des) métal (métaux) dans chaque formule donnée, et dans lequel le sodium (Na) ou le potassium (K) peuvent être substitués à l'hydrogène (H).

2. Le procédé de la revendication 1 dans lequel le catalyseur hétérogène à base d'or inclut un alliage d'or et d'au moins un métal supplémentaire sélectionné parmi le palladium, le platine, l'argent, le cuivre, le rhodium, l'iridium, le ruthénium, et des combinaisons de ceux-ci.

3. Le procédé de la revendication 1 dans lequel les conditions incluent une température comprise dans la gamme allant de 0 °C à 200 °C et une pression partielle d'alcane comprise dans la gamme allant de 1,01 x 10⁴ newtons par mètre carré à 1,42 x 10⁷ newtons par mètre carré.

4. Le procédé de la revendication 1 dans lequel l'alcane en C₁ à C₃ est sélectionné parmi le méthane, le produit oxygéné étant le méthanol ; l'éthane, le produit oxygéné étant l'éthanol ; et le propane, le produit oxygéné étant le 2-propanol.

5. Le procédé de la revendication 1 incluant en outre un promoteur sélectionné parmi le chlorure, le phosphate, le sodium, le potassium, et des combinaisons de ceux-ci.

6. Le procédé de la revendication 1 dans lequel aucun produit oxygéné carboné de l'alcane en C₁ à C₃ n'est formé.

7. Le procédé de la revendication 1 dans lequel le peroxyde d'hydrogène ou l'espèce hydroperoxy sont tout d'abord préparés en solution aqueuse et l'alcane en C₁ à C₃ entre ensuite en contact avec le peroxyde d'hydrogène ou l'espèce hydroperoxy en présence du catalyseur hétérogène à base d'or, le peroxyde d'hydrogène ou l'espèce hydroperoxy étant soit dans la solution aqueuse, soit adsorbés sur le catalyseur.

8. Le procédé de la revendication 1 dans lequel le peroxyde d'hydrogène ou l'espèce hydroperoxy sont préparés en mettant à réagir une source d'hydrogène et un gaz oxygène, en présence d'un catalyseur hétérogène à base d'or sur un support d'oxyde métallique ayant une morphologie de nanotubes, de nanofibres, de nanofils, ou de nanotiges, dans des conditions telles que du peroxyde d'hydrogène ou une espèce hydroperoxy soient formés.

9. Le procédé de la revendication 8 dans lequel la mise en réaction de la source d'hydrogène et du gaz oxygène pour former du peroxyde d'hydrogène ou une espèce hydroperoxy, et la mise en contact de l'alcane en C₁ à C₃ et du peroxyde d'hydrogène ou de l'espèce hydroperoxy, sont réalisées en étapes séquentielles dans un réacteur unique ou dans un système de réacteurs avec des étages multiples.

10. Le procédé de la revendication 9 dans lequel le réacteur unique est, ou le système de réacteurs inclut, un réacteur à lit fixe, un réacteur monolithique, un réacteur à boue, ou un réacteur à lit mobile.

11. Le procédé de la revendication 1 dans lequel soit (a) l'alcane en C₁ à C₃ et le produit oxygéné sont tous deux en phase gazeuse, et le catalyseur est solide ; soit (b) l'alcane en C₁ à C₃ est en phase gazeuse, le catalyseur est mis en suspension dans un solvant, et le produit oxygéné est en phase liquide.

12. Un procédé pour préparer du méthanol à partir de méthane comprenant les étapes séquentielles consistant à (1) mettre à réagir de l'hydrogène et du gaz oxygène pour former un premier produit comprenant du peroxyde d'hydrogène ou une espèce hydroperoxy ; et (2) mettre à réagir du méthane avec le peroxyde d'hydrogène ou l'espèce hydroperoxy pour former un deuxième produit comprenant du méthanol, à condition que les deux étapes soient réalisées en présence d'un catalyseur hétérogène à base d'or sur un support d'oxyde métallique ayant une morphologie de nanotubes, de nanofibres, de nanofils, ou de nanotiges, dans des conditions telles que le premier produit et le deuxième produit soient formés, dans lequel le support d'oxyde métallique ayant une morphologie de nanotubes, de nanofibres, de nanofils, ou de nanotiges inclut un oxyde sélectionné parmi des titanates (H_{z}Ti_{y}Oₓ), le dioxyde de titane (TiO₂), des oxydes de cérium (H_{z}Ce_{y}Oₓ), des oxydes de zirconium (H_{z}Zr_{y}Oₓ), des oxydes de vanadium (H_{z}V_{y}Oₓ), l'oxyde d'aluminium (Al₂O₃), l'oxyde de silicium (SiO₂), et des combinaisons de ceux-ci, dans lequel x représente un nombre entier de 2 à 9, y représente un nombre entier de 1 à 4, et z représente un nombre entier de 0 à 3, en fonction de la valence du (des) métal (métaux) dans chaque formule donnée, et dans lequel le sodium (Na) ou le potassium (K) peuvent être substitués à l'hydrogène (H).

13. Le procédé de la revendication 12 dans lequel, à l'étape (a) et à l'étape (b), la source d'hydrogène, le gaz oxygène, le méthane, et le peroxyde d'hydrogène ou l'espèce hydroperoxy sont tous en phase gazeuse, et le catalyseur est un solide.

14. Un procédé pour préparer du peroxyde d'hydrogène ou une espèce hydroperoxy comprenant la mise en réaction d'une source d'hydrogène et d'un gaz oxygène, en présence d'un catalyseur hétérogène à base d'or sur un support d'oxyde métallique ayant une morphologie de nanotubes, de nanofibres, de nanofils, ou de nanotiges, dans des conditions telles que du peroxyde d'hydrogène ou une espèce hydroperoxy soient formés, dans lequel le support d'oxyde métallique ayant une morphologie de nanotubes, de nanofibres, de nanofils, ou de nanotiges inclut un oxyde sélectionné parmi des titanates (H_{z}Ti_{y}Oₓ), le dioxyde de titane (TiO₂), des oxydes de cérium (H_{z}Ce_{y}Oₓ), des oxydes de zirconium (H_{z}Zr_{y}Oₓ), des oxydes de vanadium (H_{z}V_{y}Oₓ), l'oxyde d'aluminium (Al₂O₃), l'oxyde de silicium (SiO₂), et des combinaisons de ceux-ci, dans lequel x représente un nombre entier de 2 à 9, y représente un nombre entier de 1 à 4, et z représente un nombre entier de 0 à 3, en fonction de la valence du (des) métal (métaux) dans chaque formule donnée, et dans lequel le sodium (Na) ou le potassium (K) peuvent être substitués à l'hydrogène (H).
